(19) Europäisches Patentamt
European Patent Office
Office européen des brevets

(11) Veröffentlichungsnummer: **0 033 376**
**A1**

(12) # EUROPÄISCHE PATENTANMELDUNG

(21) Anmeldenummer: 80107651.4

(22) Anmeldetag: 05.12.80

(51) Int. Cl.$^3$: C 07 C 5/25
C 07 C 11/12, B 01 J 31/12
B 01 J 23/04

(30) Priorität: 02.02.80 DE 3003872

(43) Veröffentlichungstag der Anmeldung: 12.08.81 Patentblatt 81/32

(84) Benannte Vertragsstaaten: AT BE DE FR GB IT SE

(71) Anmelder: CHEMISCHE WERKE HÜLS AG
Postfach 1320
D-4370 Marl 1(DE)

(72) Erfinder: Kampf, Wolfgang, Dr.
Im Wienäckern 60 b
D-4358 Haltern(DE)

(54) **Verfahren zur Isomerisierung von isolierten zu konjugierten Doppelbindungen in Reaktionsprodukten aus konjugierten Diolefinen und aromatischen Kohlenwasserstoffen.**

(57) Verfahren zur Isomerisierung von isolierten zu konjugierten Doppelbindungen in Reaktionsprodukten aus konjugierten Diolefinen und aromatischen Kohlenwasserstoffen mit Hilfe eines Isomerisierungskatalysators und gegebenenfalls in Gegenwart eines Lösemittels, wobei man als Isomerisierungskatalysator ein Alkalialkoholat, gegebenenfalls zusammen mit einer stark polaren aprotonischen Verbindung, oder ein Umsetzungsprodukt und/oder Gemisch aus einem Alkalimetall und/oder einem Alkalihydrid und/oder einer alkalimetall- und/oder magnesiumorganischen Verbindung und Ammoniak und/oder einem Amin und/oder Hexamethylphosphorsäuretriamid eingesetzt und die Isomerisierung bei Temperaturen von 0 bis + 200 °C durchführt.

EP 0 033 376 A1

Verfahren zur Isomerisierung von isolierten zu konjugierten Doppelbindungen in Reaktionsprodukten aus konjugierten Diolefinen und aromatischen Kohlenwasserstoffen

Bei der katalytischen Umsetzung von konjugierten Diolefinen mit aromatischen Kohlenwasserstoffen werden ebenso wie bei der Homo- und Copolymerisation dieser Diene Produkte mit isolierten Doppelbindungen erhalten.

Es ist jedoch bekannt, daß nieder- und hochmolekulare Verbindungen mit konjugierten Doppelbindungen den entsprechenden Verbindungen mit isolierten Doppelbindungen an Reaktivität und damit auch an Attraktivität überlegen sind. Aus diesem Grunde wurden bereits Verfahren zur Herstellung von Verbindungen mit konjugierten Doppelbindungen entwickelt. Man kann dabei z. B. so vorgehen, daß man 1,3-Diene mit Acetylen copolymerisiert [J. Furukawa et al., Journ. Polym. Sci., Polym. Chem. Ed., Vol. 14, 1213-19 (1976)].

Eine andere und wirtschaftlichere Möglichkeit ist die Isomerisierung von isolierten zu konjugierten Doppelbindungen. Zur Durchführung dieser Isomerisierung wurden die unterschiedlichsten Katalysatorsysteme eingesetzt (DE-AS 11 74 071, DE-OS 23 42 885 sowie deutsche Patentanmeldungen P 29 24 548.5, P 29 24 577.0 und P 29 24 598.5). Bei allen diesen Verfahren wurden als Ausgangsstoffe ausschließlich niedermolekulare Homo- oder Copolymere von 1,3-Dienen eingesetzt.

Aufgabe der vorliegenden Erfindung war es nun, ein Verfahren zur Isomerisierung von isolierten zu konjugierten Doppelbindungen in Reaktionsprodukten aus konjugierten Diolefinen und aromatischen Kohlenwasserstoffen (im Nachfolgenden kurz Reaktionsprodukte genannt) zu entwickeln, um deren Anwendungsspektrum zu erweitern und vorhandene bekannte Eigenschaften zu verbessern.

Diese Aufgabe wurde überraschend durch die im Patentanspruch beschriebene Maßnahme gelöst. Überraschend deswegen, weil es angesichts der in den Molekülen vorhandenen Störung durch die Arylreste nicht zu erwarten war, daß nahezu alle in Konjugation zu bringenden aliphatischen Doppelbindungen isomerisiert werden können.

Die für das erfindungsgemäße Verfahren als Ausgangsstoffe dienenden Reaktionsprodukte aus konjugierten Diolefinen und aromatischen Kohlenwasserstoffen können nach einer Reihe von Verfahren des Standes der Technik (DE-PSS 11 37 727 und 11 70 932, US-PS 3 373 216 und JP-OS 49-32985) sowie nach einigen noch nicht zum Stand der Technik gehörenden Verfahren (deutsche Patentanmeldungen P 28 48 804.2 und P 30 00 708.0) hergestellt werden. Bei diesen Verfahren werden als konjugierte Diene z. B. Butadien, Isopren, Piperylen und 2,3-Dimethylbutadien eingesetzt, wobei Butadien bevorzugt ist.

Mit den konjugierten Dienen zur Reaktion gebracht werden im allgemeinen aromatische Kohlenwasserstoffe, die unter den jeweiligen Reaktionsbedingungen keine oder nur geringe unerwünschte Nebenreaktionen verursachen, z. B. Benzol, Toluol, Xylole, Cumol oder Alkenylaromaten, wie Styrol und Butenylbenzol.

Selbstverständlich können auch Mischungen eingesetzt werden, ebenfalls solche, die neben dem (den) reaktiven aromatischen Kohlenwasserstoff(en) solche Kohlenwasserstoffe enthalten, die unter den angewandten Bedingungen nicht mit dem Dien reagieren, wie z. B. Hexan, Heptan, Octan und Cyclohexan.

Im allgemeinen wird das konjugierten Dien in einer Konzentration von 1 bis 50 Gewichtsprozent, bezogen auf den aromatischen Kohlenwasserstoff, eingesetzt. Der Konzentrationsbereich von 5 bis 40 Gewichtsprozent ist bevorzugt.

Natürlich können bei dem erfindungsgemäßen Verfahren nur Reaktionsprodukte mit mindestens zwei aliphatischen Doppelbindungen im Molekül im gewünschten Sinne isomerisiert werden.

Als Isomerisierungskatalysatoren können bei dem erfindungsgemäßen Verfahren die im Patentanspruch unter 1 und 2 beschriebenen Verbindungen bzw. Systeme eingesetzt werden.

Darunter fallen zunächst (1) Alkalialkoholate der allgemeinen Formel $R^1OMe$, in der $R^1$ ein geradkettiger oder verzweigter gesättigter oder ungesättigter (cyclo-)aliphatischer Rest mit bis zu 20 Kohlenstoffatomen, vorzugsweise 3 bis 8 Kohlenstoffatomen, und Me vorzugsweise Natrium oder Kalium sein können. Weiterhin geeignet sind Alkalialkoholate mehrwertiger Alkohole der oben angegebenen C-Zahl. Alle den Alkoholaten zugrundeliegenden Alkohole können neben der Alkoholfunktion noch andere funktionelle Gruppen, wie z. B. Ether- oder Aminfunktion, tragen. Bi- oder mehrfunktionelle Alkohole sind beispielsweise gegebenenfalls einseitig veretherte Polyethylenglykole.

Typische einsetzbare Alkoholate sind z. B. Natrium- und Kaliumisopropylat, Natrium- und Kalium-tert.-butylat, Natrium- und Kalium-2-ethyl-hexylat.

Damit beim Einsatz von Alkalialkoholaten unter schonenderen Temperaturbedingungen gearbeitet werden kann, können zusätzlich stark polare aprotonische Substanzen, wie z. B. Dimethylsulfoxid, Dimethylformamid und N-Methylpyrrolidon, eingesetzt werden.

Das Alkalialkoholat wird im allgemeinen dem zu isomerisierenden Reaktionsprodukt in einer Menge von 0,1 bis 10, vorzugsweise 0,5 bis 3 Gramm, pro 100 Gramm Reaktionsprodukt zugesetzt. Beim Einsatz einer stark polaren

aprotonischen Substanz beträgt deren Menge im allgemeinen bis zu 20 Gramm, vorzugsweise 1 bis 10 Gramm, pro 100 Gramm Reaktionsprodukt.

Weitere einsetzbare Isomerisierungskatalysatoren (2) sind die Umsetzungsprodukte oder Gemische aus einem Alkalimetall und/oder einem Alkalihydrid und/oder einer alkalimetall- oder magnesiumorganischen Verbindung und Ammoniak, einem Amin oder Hexamethylphosphorsäuretriamid.

Als Alkalimetalle kommen z. B. Lithium, Natrium und Kalium, vorzugsweise Natrium, als Alkalihydride Lithium-, Natrium- und Kaliumhydrid, vorzugsweise Natriumhydrid, und als alkalimetallorganische Verbindungen solche der allgemeinen Formel $R^2Me$, in der Me für Lithium, Natrium oder Kalium, vorzugsweise Lithium und Natrium, und $R^2$ für eine Alkyl-, Alkenyl-, Cycloalkyl-, Aryl- oder Aralkylgruppe mit 1 bis 20 Kohlenstoffatomen, vorzugsweise 1 bis 7 Kohlenstoffatomen, steht, infrage. Bevorzugte alkalimetallorganische Verbindungen sind Lithiumbutyl und Natriumamyl. Als magnesiumorganische Verbindungen können solche der allgemeinen Formel $R^3_y$-Mg-$X_z$, mit $R^3$ gleich Alkyl ($C_1$ bis $C_8$), X gleich Chlor oder Brom, y gleich 0,5 bis 2,0 und z gleich 2-y eingesetzt werden. Typische Vertreter sind Ethylmagnesiumbromid und Propylmagnesiumchlorid.

Geeignete Amine sind (cyclo-)aliphatische und aromatische Mono- und Polyamine mit 1 bis 20 Kohlenstoffatomen, vorzugsweise 1 bis 8 Kohlenstoffatomen. Bevorzugt sind aliphatische Polyamine mit 2 bis 4 Stickstoffatomen, wie z. B. Tetramethylethylendiamin, Dipropylentriamin und Bis-(3-aminopropyl)-ethylendiamin.

Das molare Verhältnis von Alkalimetall bzw. von Metallverbindung zu den genannten stickstoffhaltigen Verbindungen beträgt im allgemeinen 5 : 1 bis 1 : 20, vorzugsweise 2 : 1 bis 1 : 10. Ein Überschuß von stickstoffhaltiger Verbindung ist nicht nur beim Einsatz eines Gemisches der Katalysatorkomponenten von Vorteilen, sondern auch beim Einsatz der Umsetzungsprodukte (Amide), vor allem bei niedrigen Katalysatorkonzentrationen, wie z. B. 2 Milligrammatom Alkali pro 100 g zu isomerisierendem Reaktionsprodukt.

Die verschiedenen beim erfindungsgemäßen Verfahren einsetzbaren Katalysatoren bzw. Katalysatorsysteme (2) werden im allgemeinen dem zu isomerisierenden Reaktionsprodukt in einer solchen Menge zugesetzt, daß pro 100 g 1 bis 100 Milligrammatom, vorzugsweise 5 bis 40 Milligrammatom, Alkalimetall bzw. Magnesium vorhanden sind. Auf diese Weise lassen sich die konjugierbaren Anteile, d. h. die mindestens 2 aliphatische Doppelbindungen pro Molekül enthaltenden Reaktionsprodukte, bis zu 100 % isomerisieren. Die zu isomerisierenden Reaktionsprodukte können sowohl mit als auch ohne Lösemittel eingesetzt werden.

Als Lösemittel, die frei von störenden Verunreinigungen, wie z. B. Sauerstoff und Wasser, sein sollen, kommen z. B. aliphatische, cycloaliphatische und aromatische Kohlenwasserstoffe infrage. Typische Vertreter aus diesen Gruppen sind Hexan, Octan, Cyclohexan, Benzol und Toluol.

Das erfindungsgemäße Verfahren wird im allgemeinen bei Temperaturen von 0 bis +200 °C durchgeführt. Bevorzugt ist der Bereich von +20 bis +180 °C, besonders bevorzugt ist der Bereich von +50 bis +150 °C.

Bei der Durchführung des erfindungsgemäßen Verfahrens geht man im allgemeinen so vor, daß man das zu isomerisierende Reaktionsprodukt, gegebenenfalls gelöst in einem Lösemittel, nach Zusatz des Katalysators bzw. der Katalysatorkomponenten gegebenenfalls unter Rühren auf die gewünschte Temperatur bringt.

Die erforderliche Reaktionszeit richtet sich nach Art und

Menge des Katalysators bzw. der Katalysatorkomponenten und nach der Temperatur. Die optimale Reaktionszeit läßt sich durch einige orientierende Versuche leicht ermitteln. Die Reaktion wird durch Zugabe einer H-aciden Verbindung, wie z. B. Methanol oder Isopropanol, beendet. Bis zu diesem Verfahrensschritt werden alle Operationen, besonders bei höheren Temperaturen, unter Schutzgasatmosphäre, vorzugsweise unter Argon, durchgeführt. Anschließend wird der Katalysator durch wäßrige Wäsche und/oder eine adsorptive Behandlung, z. B. mit Bleicherde, weitestgehend entfernt.

Die nach dem erfindungsgemäßen Verfahren isomerisierten Reaktionsprodukte, die im allgemeinen ein zahlenmittleres Molekulargewicht ($\bar{M}n$) von ca. 150 bis 500, vorzugsweise 200 bis 300, haben, können z. B. als Dienkomponenten bei Diels-Alder-Additionen mit z. B. Vinylsilanen verwendet werden. Solche Additionsprodukte können als Haftvermittler bei der Herstellung von Polymer-Füllstoff-Mischungen eingesetzt werden.

Das erfindungsgemäße Verfahren wird durch die nachfolgenden Beispiele näher erläutert.

<u>Beispiel 1</u>

500 g eines nach dem Verfahren der deutschen Patentanmeldung P 28 48 804.2 hergestellten, dünnflüssigen Reaktionsproduktes wurden zusammen mit 31,5 ml 2molarer Lithiumbutyl-Lösung (LiBu) in Hexan und 22,5 ml Dipropylentriamin (≙ 157,5 mMol) in einen ausgeheizten und mehrfach mit Argon gespülten 1 l-Rührkolben gegeben. Das Ausgangsprodukt hatte folgende Kenndaten:

| | | |
|---|---|---|
| $^1$H-NMR | 23,4 % | aromatischer Wasserstoff |
| | 11,3 % | olefinischer Wasserstoff |
| | 7,0 % | benzylischer Wasserstoff |
| | 58,3 % | aliphatischer Wasserstoff |

$\overline{M}n$ 240
IR (Absolutwerte) Doppelbindungsverteilung:
trans-1,4 13 %
1,2 0,7 %
Viskosität ≤ 30 mPas
Resttoluolgehalt: 4,36 Gew.-% (gaschr. bestimmt)
Daraus errechnet sich ein Polybutadienanteil von 57,4 %. Der Konjuengehalt lag bei 0,4 %. Aus den Angaben folgt weiterhin, daß im Mittel in 2 Molekülen 3 Doppelbindungen enthalten sind.

Der durch die Zugabe von LiBu und Amin weinrot gefärbte Ansatz wurde unter Argonatmosphäre bei ständigem Rühren auf 80 °C erwärmt. Dabei verfärbte sich der Kolbeninhalt nach Dunkelrot bis Schwarz. Nach 1 Stunde Reaktionszeit wurden 4,8 ml Isopropanol zur Beendigung der Reaktion zugegeben.

Zur Aufarbeitung wurden zunächst 500 ml Hexan zugegeben. Danach wurde 60 Minuten lang mit 20 g Bleicherde bei Zimmertemperatur verrührt und die Bleicherde durch Zentrifugieren abgetrennt. Nach zweimaligem Durchlauf durch einen Dünnschichtverdampfer bei 120 °C im Wasserstrahlvakuum erfolgte ein weiterer Durchlauf bei 120 °C im Ölpumpenvakuum. Nach erneutem Zentrifugieren wurden 380 g eines Produktes gewonnen, das nach UV-spektroskopischer Untersuchung 17,6 % konjugierte Diolefine, berechnet als Gewichtsprozent $C_8H_{14}$, enthielt. Aus obigen Angaben läßt sich ableiten, daß somit alle zur Konjugation möglichen Anteile, d. h. alle Moleküle mit mindestens 2 Doppelbindungen, konjugiert wurden. Der Li-Gehalt war < 0,5 ppm.

Beispiele 2 bis 19

Bei analoger Verfahrensweise wie in Beispiel 1 beschrieben wurden Versuche unter veränderten Versuchsbedingungen und mit anderen Katalysatorsystemen durchgeführt. Die Abkürzungen bedeuten:

LiBu = Lithiumbutyl
KTBO = Kalium-tert.-butylat
TMED = Tetramethylethylendiamin
DPTA = Dipropylentriamin
DMSO = Dimethylsulfoxid

Bei den Versuchen kamen zwei Ausgangsprodukte zum Einsatz (A und B).

A hatte folgende Kenndaten:

$^1H$-NMR 23,4 % aromatischer Wasserstoff
11,3 % olefinischer Wasserstoff
7,0 % benzylischer Wasserstoff
58,3 % aliphatischer Wasserstoff
IR (Absolutwerte) Doppelbindungsverteilung:
trans-1,4 13 %
1,2 0,7 %
cis-1,4 nicht auswertbar
$\bar{M}n$ 240
Polybutadienanteil 57,4 %
Viskosität ≤ 30 mPas
berechneter maximal erreichbarer Konjuengehalt: 17,6 %
Resttoluolgehalt: 4,36 Gew.-% (gaschr. bestimmt)

B hatte folgende Kenndaten:

$^1H$-NMR 18,3 % aromatischer Wasserstoff
11,0 % olefinischer Wasserstoff
4,8 % benzylischer Wasserstoff
65,9 % aliphatischer Wasserstoff
IR (Absolutwerte) Doppelbindungsverteilung:
trans-1,4 16 %
1,2 1 %
cis-1,4 nicht auswertbar
$\bar{M}n$ 237
Polybutadienanteil 65,2 %
Viskosität 130 mPas

Jodzahl 157
berechneter maximal erreichbarer Konjuengehalt: 17,1 %
Resttoluolgehalt: 2,16 Gew.-% (gaschr. bestimmt)

Beide Ausgangsprodukte hatte < 0,5 % Konjuengehalt. Die Polybutadienanteile wurden jeweils aus den Ergebnissen der $^1$H-Kernresonanzuntersuchungen berechnet. Letztere und die $\overline{M}$n-Werte dienten als Berechnungsgrundlagen für den angegebenen maximal erreichbaren Konjuengehalt.

Die Konjuengehalte wurden durch Auswertung der Absorptionsbande bei 230 nm ermittelt (UV-Spektren). Sie sind als Gewichtsprozent, berechnet als $C_8H_{14}$, angegeben.

Tabelle 1

| Bsp. Nr. | Ausgangs-produkt | Katalysator | | | | Tempe-ratur [°C] | Zeit [h] | konjugierte Diolefine [%] |
|---|---|---|---|---|---|---|---|---|
| | | Metallverbindung | | Amin | | | | |
| | | Art | Menge [g/100 g A/B] | Art | Menge [g/100 g A/B] | | | |
| 2 | A | LiBu | 0,53 | TMED | 2,44 | 80 | 3 | 2,1 |
| 3 | A | LiBu | 1,33 | TMED | 6,10 | 80 | 4 | 14,9 |
| 4 | B | LiBu | 0,27 | DPTA | 1,4 | 80 | 5 | 5,0 |
| 5 | B | LiBu | 0,53 | DPTA | 2,8 | 80 | 3 | 11,6 |
| 6 | B | LiBu | 1,33 | DPTA | 7,0 | 80 | 1 | 11,8 |
| 7 | B | LiBu | 2,66 | DPTA | 14,0 | 80 | 0,5 | 10,0 |
| 8 | B | LiBu | 0,53 | DPTA | 2,8 | 50 | 3 | 9,1 |
| 9 | B | LiBu | 0,53 | DPTA | 2,8 | 20 | 5 | 8,2 |
| 10 | B | NaH | 0,50 | DPTA | 7 | 80 | 5 | 6,6 |
| 11 | B | NaH | 1,00 | DPTA | 14 | 80 | 5 | 8,0 |
| 12 | B | $NaNH_2$ | 0,81 | DPTA | 7 | 80 | 5 | 7,5 |
| 13 | B | $NaNH_2$ | 1,63 | DPTA | 14 | 80 | 5 | 9,3 |

0033376

Tabelle 2

| Bsp. Nr. | Ausgangs-produkt | Katalysator | | | | Tempe-ratur [°C] | Zeit [h] | konjugierte Diolefine [%] |
|---|---|---|---|---|---|---|---|---|
| | | Metallverbindung | | Zusatz | | | | |
| | | Art | Menge [g/100 g A/B] | Art | Menge [g/100 g A/B] | | | |
| 14 | A | KTBO | 1 | - | - | 120 | 4 | 16,3 |
| 15 | A | KTBO | 2,5 | - | - | 120 | 3 | 17,6 |
| 16 | B | KTBO | 1 | DMSO | 5 | 80 | 3 | 10,4 |
| 17 | B | KTBO | 2,5 | DMSO | 5 | 80 | 5 | 11,5 |
| 18 | A | KTBO | 1 | DMSO | 5 | 80 | 3 | 17,1 |
| 19 | A | KTBO | 2,5 | DMSO | 5 | 80 | 3 | 15,3 |

Jl

0033376
O.Z. 3623

Patentansprüche:

1. Verfahren zur Isomerisierung von isolierten zu konjugierten Doppelbindungen in Reaktionsprodukten aus konjugierten Diolefinen und aromatischen Kohlenwasserstoffen mit Hilfe eines Isomerisierungskatalysators und gegebenenfalls in Gegenwart eines Lösemittels, dadurch gekennzeichnet, daß man als Isomerisierungskatalysator

 1 ein Alkalialkoholat, gegebenenfalls zusammen mit

  1.1 einer stark polaren aprotonischen Verbindung, oder

 2 ein Umsetzungsprodukt und/oder Gemisch aus

  2.1 einem Alkalimetall und/oder einem Alkalihydrid und/oder einer alkalimetall- und/oder magnesiumorganischen Verbindung und

  2.2 Ammoniak und/oder einem Amin und/oder Hexamethylphosphorsäuretriamid einsetzt.

2. Verfahren nach Anspruch 1, dadurch gekennzeichnet, daß man als Katalysatorkomponente 1.1 Dimethylsulfoxid, Dimethylformamid oder N-methylpyrrolidon verwendet.

Europäisches Patentamt

# EUROPÄISCHER RECHERCHENBERICHT

Nummer der Anmeldung
EP 80 10 7651.4

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| E,D | DE - A1 - 2 924 577 (CHEMISCHE WERKE HÜLS)<br>* Anspruch 1 *<br>-- | 1 |
| | DE - A - 2 329 000 (FIRESTONE TIRE & RUBBER CO.)<br>* Ansprüche 1 bis 4 *<br>-- | 1,2 |
| P | US - A - 4 205 192 (T. HARADA)<br>* Ansprüche 1, 2 *<br>-- | 1 |
| | US - A - 3 501 540 (E.A. ZUECH)<br>* Anspruch 1 *<br>-- | 1,2 |
| A | US - A - 3 432 498 (A.N. KURTZ et al.)<br>* Ansprüche 1, 2 *<br>-- | 1,2 |
| A | Chemical Abstracts Band 80, Nr. 25, 1974<br>Columbus, Ohio, USA<br>Y. FUJITA et al. "Alkylidenenorbornenes"<br>Seite 398, Spalte 1, Abstract Nr. 145587h<br>& JP - A - 74 - 07258<br>-- | 1,2 |
| A | Chemical Abstracts Band 85, Nr. 17,1976<br>Columbus, Ohio, USA<br>H. KAWAUCHI et al. "Isomerizing 5-vinyl norbornene"<br>Seite 602, Spalte 1, Abstract Nr. 123438m<br>& JP - A - 75 - 35072<br>---- | 1 |

KLASSIFIKATION DER ANMELDUNG (Int. Cl.³)

C 07 C 5/25
C 07 C 11/12
B 01 J 31/12
B 01 J 23/04

RECHERCHIERTE SACHGEBIETE (Int. Cl.³)

B 01 J 23/04
B 01 J 31/12
C 07 C 5/25
C 07 C 11/12

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 14-05-1981 | KNAACK |